# EUROPEAN PATENT SPECIFICATION

(11) **EP 4 020 486 B1**
(45) Date of publication and mention of the grant of the patent: **12.11.2025**
(21) Application number: 21211166.0
(22) Date of filing: 29.11.2021
(51) Int. Cl.: G16H 10/60

(54) **SYSTEM AND METHOD FOR PROVIDING HEALTH STATUS WITH AN EVENT TICKET**
SYSTEM UND VERFAHREN ZUR BEREITSTELLUNG DES GESUNDHEITSSTATUS MIT EINEM VERANSTALTUNGSTICKET
SYSTÈME ET PROCÉDÉ POUR FOURNIR UN ÉTAT DE SANTÉ AVEC UN BILLET D'ÉVÉNEMENT

(30) Priority: 22.12.2020 US 202063128979 P
(43) Date of publication of application: 29.06.2022
(73) Proprietor: BlackBerry Limited, Waterloo, Ontario N2K 0A7 (CA)
(72) Inventor: BARRETT, Stephen John, Waterloo, N2K 0A7 (CA); ALFRED, James Robert, Waterloo, N2K 0A7 (CA); TSANG, Ming Chee, Waterloo, N2K 0A7 (CA); MONTEMURRO, Michael Peter, Waterloo, N2K 0A7 (CA); SCHIEMAN, Adam Richard, Waterloo, N2K 0A7 (CA); ADAMS, Neil Patrick, Waterloo, N2K 0A7 (CA)
(74) Representative: Murgitroyd & Company

(56) References cited:
- WO-A1-2011/005224
- AU-A4- 2020 101 208

## Description

### TECHNICAL FIELD

The present disclosure relates to systems and methods for providing health status with an event ticket.

### BACKGROUND

Businesses and other establishments may wish to check the health status of a visitor or guest prior to allowing them to enter. For example, a business may wish to ensure that the visitor does not have a particular virus or disease prior to allowing the visitor to enter.

In some instances, a business may check the temperature of a visitor prior to allowing the visitor to enter. Taking the temperature of the visitor may not accurately detect whether or not the visitor has a particular virus or disease.
In some instances, a business may check the vaccination records of a visitor prior to allowing the visitor to enter. Checking vaccination records may be inaccurate as the vaccination records may be out of date. It may also be difficult to determine that the vaccination records are in fact the vaccination records of the visitor or if they are fraudulent in that the vaccination records are that of another person.
WO 2011/005224 A1 discloses a system in which a user inputs or updates his personal data and completes through a web site a health declaration by answering questions.
AU 2020 101 208 A4 discloses a care management system for verifying and validating pathogen care events associated with a patient.

### SUMMARY

Accordingly there is provided a method, a system, and a computer program as detailed in the claims that follow.

### BRIEF DESCRIPTION OF THE DRAWINGS

Embodiments are described in detail below, with reference to the following drawings:
FIG. 1 is a schematic operation diagram illustrating an operating environment of an example embodiment;
FIG. 2 is a simplified schematic diagram showing components of a computing device;
FIG. 3 is a high-level schematic diagram of an example computer device;
FIG. 4 shows a simplified organization of software components stored in a memory of the example computer device of FIG. 3; and
FIG. 5 is a flowchart showing operations performed in generating a certificate signing request, according to an embodiment;
FIG. 6 shows an example image of a user and a corresponding obscured image of the user, according to an embodiment;
FIG. 7 shows another example image of a user and corresponding obscured images of the user, according to an embodiment;
FIG. 8 is a flowchart showing operations performed in completing a certificate signing request, according to an embodiment;
FIG. 9 is a flowchart showing operations performed in obtaining a signed certificate, according to an embodiment;
FIG. 10 is a flowchart showing operations performed in displaying an image of a user, according to an embodiment;
FIG. 11 is an example image of a user including a nonce, according to an embodiment;
FIG. 12 is an example corresponding obscured image of a user including a nonce, according to an embodiment;
FIG. 13 is a flowchart showing operations performed in obtaining a signed certificate, according to an embodiment;
FIG. 14 is a flowchart showing operations performed in signing a certificate according to an embodiment; and
FIG. 15 is a flowchart showing operations performed in signing a certificate, according to an embodiment;
FIG. 16 is a flowchart showing operations performed in generating a scannable code;
FIG. 17 is an exemplary display screen displayed by a relying party device in response to scanning a scannable code; and
FIG. 18 is another exemplary display screen displayed by a relying party device in response to scanning a scannable code.

Like reference numerals are used in the drawings to denote like elements and features.

### DETAILED DESCRIPTION OF VARIOUS EMBODIMENTS

Accordingly, in one aspect there is provided a computer-implemented method comprising obtaining data associated with an event ticket and data associated with a digital health certificate of a user; generating a scannable code that includes the data associated with the event ticket and the data associated with the digital health certificate of the user, the scannable code configured to be scanned by a relying party device and causing the relying party device to display at least a notification indicating a ticket status and information associated with the user; and providing the scannable code to memory of a user device.

In one or more embodiments, the data associated with the digital health certificate of the user includes partial personally identifiable information of the user.

In one or more embodiments, the data associated with the digital health certificate includes information indicating a health status of the user.

In one or more embodiments, the health status of the user includes at least one of a vaccination record, an immunisation status, a start date of immunisation status, an end date of immunisation status, or an indication as to whether or not the user has a particular disease.

In one or more embodiments, the scannable code, when scanned by the relying party device, causes the relying party device to send, to a server associated with a ticket service, a signal requesting ticket status; receive, from the server associated with the ticket service, a signal indicating the ticket status; and display, on a display screen of the relying party device, the ticket status, the health status of the user and the partial personally identifiable information of the user.

In one or more embodiments, the scannable code, when scanned by the relying party device, causes the relying party device to send, to a server associated with a ticket service, a signal requesting ticket status; receive, from the server associated with the ticket service, a signal indicating the ticket status; and display, on a display screen of the relying party device, the ticket status and the partial personally identifiable information of the user.

In one or more embodiments, the method further comprises receiving, at the relying party device, information indicating health status of the user, the information indicating health status of the user provided by the user device.

In one or more embodiments, the data associated with the digital health certificate includes a single use health status code and the scannable code is configured to cause the relying party device to obtain health status information of the user based on the single use health status code.

In one or more embodiments, the relying party device obtains the health status information of the user based on the single use health status code from one of a ticketing server or an application server.

In one or more embodiments, the scannable code is a quick response (QR) code.

According to another aspect there is provided a system comprising at least one processor; and a memory coupled to the at least one processor and storing instructions that, when executed by the at least one processor, configure the at least one processor to obtain data associated with an event ticket and data associated with a digital health certificate of a user; generate a scannable code that includes the data associated with the event ticket and the data associated with the digital health certificate of the user, the scannable code configured to be scanned by a relying party device and causing the relying party device to display at least a notification indicating a ticket status and information associated with the user; and provide the scannable code to memory of a user device.

In one or more embodiments, the data associated with the digital health certificate of the user includes partial personally identifiable information of the user.

In one or more embodiments, the data associated with the digital health certificate includes information indicating a health status of the user.

In one or more embodiments, the health status of the user includes at least one of a vaccination record, an immunisation status, a start date of immunisation status, an end date of immunisation status, or an indication as to whether or not the user has a particular disease.

In one or more embodiments, the scannable code, when scanned by the relying party device, causes the relying party device to send, to a server associated with a ticket service, a signal requesting ticket status; receive, from the server associated with the ticket service, a signal indicating the ticket status; and display, on a display screen of the relying party device, the ticket status and information associated with the user, the information associated with the user including the health status of the user and the partial personally identifiable information of the user.

In one or more embodiments, the scannable code, when scanned by the relying party device, causes the relying party device to send, to a server associated with a ticket service, a signal requesting ticket status; receive, from the server associated with the ticket service, a signal indicating the ticket status; and display, on a display screen of the relying party device, the ticket status and information associated with the user, the information associated with the user including the partial personally identifiable information of the user.

In one or more embodiments, the relying party device receives, from the user device, information indicating health status of the user.

In one or more embodiments, the data associated with the digital health certificate includes a single use health status code and the instructions, when executed by the at least one processor, further configure the at least one processor to generate the scannable code to include the single use health status code, the scannable code configured to be scanned by the relying party device and causing the relying party device to obtain the health status information of the user based on the single use health status code.

In one or more embodiments, the relying party device obtains the health status information of the user based on the single use health status code from one of a ticketing server or an application server.

According to another aspect there is provided a non-transitory computer readable storage medium comprising computer-executable instructions which, when executed, configure a processor to obtain data associated with an event ticket and data associated with a digital health certificate of a user; generate a scannable code that includes the data associated with the event ticket and the data associated with the digital health certificate of the user, the scannable code configured to be scanned by a relying party device and causing the relying party device to display at least a notification indicating a ticket status and information associated with the user; and provide the scannable code to memory of a user device.

Other aspects and features of the present application will be understood by those of ordinary skill in the art from a review of the following description of examples in conjunction with the accompanying figures.

In the present application, the term "and/or" is intended to cover all possible combinations and sub-combinations of the listed elements, including any one of the listed elements alone, any sub-combination, or all of the elements, and without necessarily excluding additional elements.

In the present application, the phrase "at least one of ...or..." is intended to cover any one or more of the listed elements, including any one of the listed elements alone, any sub-combination, or all of the elements, without necessarily excluding any additional elements, and without necessarily requiring all of the elements.

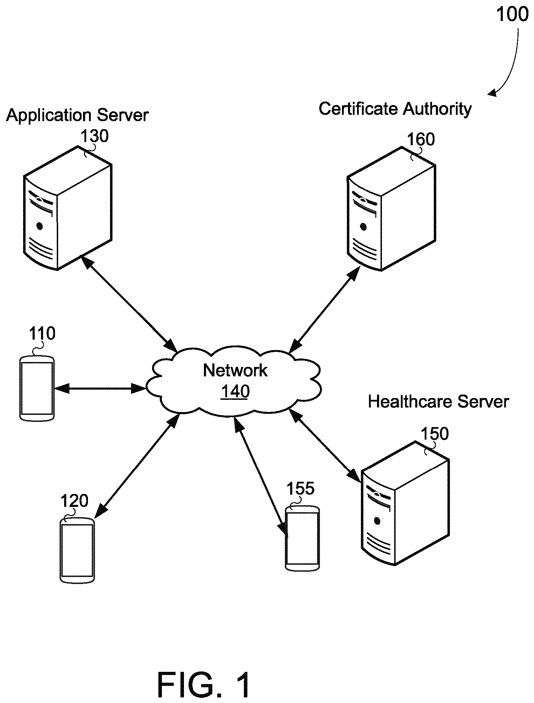

FIG. 1 is a schematic operation diagram illustrating an operating environment of an example embodiment.

As shown, mobile devices 110, 120 and a server 130 communicate via a network 140. The mobile device 110 may be referred to as a user device 110 and the mobile device 120 may be referred to as a relying party device 120.

The server 130 may be referred to as an application server 130. The application server 130 is associated with a mobile application (such as a web or mobile application) that is resident on the user device 110 and/or the relying party device 120. The mobile application may be, for example, a health passport application. As will be described in more detail below, the health passport application may be configured to submit a request for a signed certificate that includes health status data for a user and the server 130 may be used to store one or more signed certificates in memory thereof.

The system 100 also includes a healthcare data server 150 and a healthcare communication device 155. The healthcare data server 150 and the healthcare communication device 155 may be associated with one or more healthcare providers and may be configured to generate health status data for a user. The healthcare data server 150 and/or the healthcare communication device 155 may communicate with one or more external servers that may provide data such as test result data to the healthcare data server 150. The healthcare data server 150 and/or the healthcare communication device 155 may receive a certificate signing request from the user device 110 and may generate or otherwise store information related to the health status of the user. The test result data may be included with the health status data. The health status data for the user may be generated by an operator associated with the healthcare provider. An operator of the healthcare communication device 155 may be for example a physician and the user may be a patient of the physician.

As will be described, the healthcare data server 150 and/or the healthcare communication device 155 may be configured to receive image data of the user from the user device 110. The image data of the user may include an image of the user and a corresponding obscured image of the user.

The healthcare data server 150 and/or the healthcare communication device 155 may be a registration authority (RA) which generates a certificate signing request.

The system 100 also includes a certificate authority server 160. The certificate authority server 160 may be associated with an authenticating entity and may be configured to receive a certificate signing request from the healthcare data server 150 and/or the healthcare communication device 155. As will be described in more detail, certificate signing may require verifying that the registration authority (RA) is authorized and authentic. The certificate authority server 160 may include a server comprising signed certificates provided by a certificate authority or a server managed by a certificate authority.

When it is determined that the RA is authorized and authentic, the certificate authority server 160 may generate the requested certificate. The certificate may include the certificate identification number, the public key of the user, a hash of an obscured photo of the user, and health status data of the user. For example, the certificate may be an immunisation certificate and as such may include the immunisation status of the user (immune/not immune), a start date and an end date of the validity of the immunisation, and the disease that the user is immune to (for example, Covid-19). As another example, the certificate may be a vaccination certificate and as such may include information indicating that the user has been vaccinated for one or more particular diseases.

The user device 110, the relying party device 120, the application server 130, the healthcare data server 150 and the certificate authority server 160 may be in geographically disparate locations. Put differently, the user device 110, the relying party device 120, the application server 130, the healthcare data server 150 and the certificate authority server 160 may be remote from one another.

The user device 110, the relying party device 120, the application server 130, the healthcare data server 150, the healthcare communication device 155 and the certificate authority server 160 are computer systems. The user device 110, the relying party device 120 and/or the healthcare communication device 155 may take a variety of forms including, for example, a mobile communication device such as a smartphone, a tablet computer, a wearable computer such as a head-mounted display or smartwatch, a laptop or desktop computer, or a computing device of another type.

The network 140 is a computer network. In some embodiments, the network 140 may be an internetwork such as may be formed of one or more interconnected computer networks. For example, the network 140 may be or may include an Ethernet network, a wireless network, a cloud network, a telecommunications network or the like.

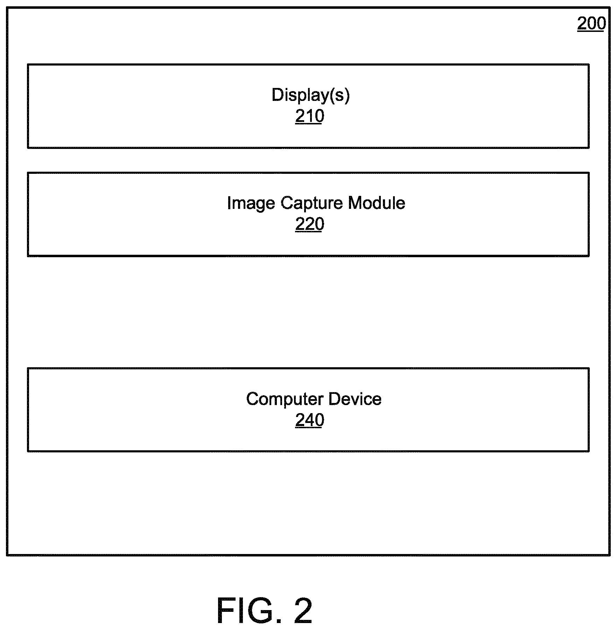

FIG. 2 is a simplified schematic diagram showing components of an exemplary computing device 200. The user device 110, the relying party device 120 and/or the healthcare communication device 155 may be of the same type as computing device 200. The computing device 200 may include modules including, as illustrated, for example, one or more displays 210, an image capture module 220, and a computer device 240.

The one or more displays 210 are a display module. The one or more displays 210 may be used to display screens of a graphical user interface that may be used, for example, to communicate with the application server 130 and/or the healthcare data server 150 (FIG. 1). The one or more displays 210 may be internal displays of the computing device 200 (e.g., disposed within a body of the computing device).

The image capture module 220 may be or may include a camera. The image capture module 220 may be used to obtain image data, such as images. The image capture module 220 may be or may include a digital image sensor system as, for example, a charge coupled device (CCD) or a complementary metal-oxide-semiconductor (CMOS) image sensor.

The computer device 240 is in communication with the one or more displays 210, and the image capture module 220. The computer device 240 may be or may include a processor which is coupled to the one or more displays 210 and/or the image capture module 220.

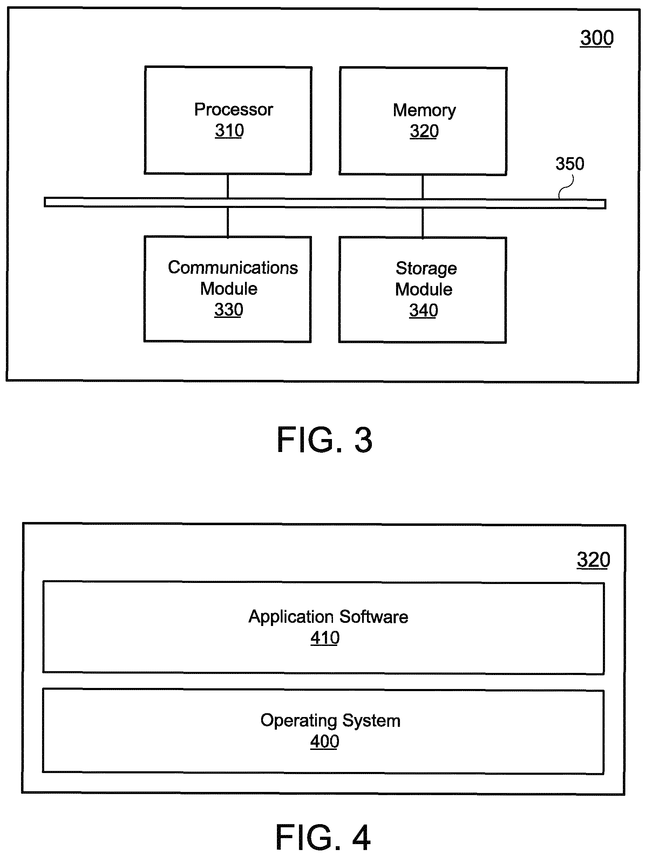

Referring now to FIG. 3, a high-level operation diagram of an example computer device 300 is shown. In some embodiments, the computer device 300 may be exemplary of the computer device 240 (FIG. 2), the application server 130, the healthcare data server 150 and/or the certificate authority server 160.

The example computer device 300 includes a variety of modules. For example, as illustrated, the example computer device 300 may include a processor 310, a memory 320, a communications module 330, and/or a storage module 340. As illustrated, the foregoing example modules of the example computer device 300 are in communication over a bus 350.

The processor 310 is a hardware processor. The processor 310 may, for example, be one or more ARM, Intel x86, PowerPC processors or the like.

The memory 320 allows data to be stored and retrieved. The memory 320 may include, for example, random access memory, read-only memory, and persistent storage. Persistent storage may be, for example, flash memory, a solid-state drive or the like. Read-only memory and persistent storage are a non-transitory computer-readable storage medium. A computer-readable medium may be organized using a file system such as may be administered by an operating system governing overall operation of the example computer device 300.

The communications module 330 allows the example computer device 300 to communicate with other computer or computing devices and/or various communications networks. For example, the communications module 330 may allow the example computer device 300 to send or receive communications signals. Communications signals may be sent or received according to one or more protocols or according to one or more standards. For example, the communications module 330 may allow the example computer device 300 to communicate via a cellular data network, such as for example, according to one or more standards such as, for example, Global System for Mobile Communications (GSM), Code Division Multiple Access (CDMA), Evolution Data Optimized (EVDO), Long-term Evolution (LTE) or the like. Additionally or alternatively, the communications module 330 may allow the example computer device 300 to communicate using near-field communication (NFC), via Wi-Fi (TM), using Bluetooth (TM) or via some combination of one or more networks or protocols. In some embodiments, all or a portion of the communications module 330 may be integrated into a component of the example computer device 300. For example, the communications module may be integrated into a communications chipset. In some embodiments, the communications module 330 may be omitted such as, for example, if sending and receiving communications is not required in a particular application.

The storage module 340 allows the example computer device 300 to store and retrieve data. In some embodiments, the storage module 340 may be formed as a part of the memory 320 and/or may be used to access all or a portion of the memory 320. Additionally or alternatively, the storage module 340 may be used to store and retrieve data from persisted storage other than the persisted storage (if any) accessible via the memory 320. In some embodiments, the storage module 340 may be used to store and retrieve data in a database. A database may be stored in persisted storage. Additionally or alternatively, the storage module 340 may access data stored remotely such as, for example, as may be accessed using a local area network (LAN), wide area network (WAN), personal area network (PAN), and/or a storage area network (SAN). In some embodiments, the storage module 340 may access data stored remotely using the communications module 330. In some embodiments, the storage module 340 may be omitted and its function may be performed by the memory 320 and/or by the processor 310 in concert with the communications module 330 such as, for example, if data is stored remotely. The storage module may also be referred to as a data store.

Software comprising instructions is executed by the processor 310 from a computer-readable medium. For example, software may be loaded into random-access memory from persistent storage of the memory 320. Additionally or alternatively, instructions may be executed by the processor 310 directly from read-only memory of the memory 320.

FIG. 4 depicts a simplified organization of software components stored in the memory 320 of the example computer device 300 (FIG. 3). As illustrated, these software components include an operating system 400 and an application 410.

The operating system 400 is software. The operating system 400 allows the application 410 to access the processor 310 (FIG. 3), the memory 320, and the communications module 330 of the example computer device 300 (FIG. 3). The operating system 400 may be, for example, Google (TM) Android (TM), Apple (TM) iOS (TM), UNIX (TM), Linux (TM), Microsoft (TM) Windows (TM), Apple OSX (TM) or the like.

The application 410 adapts the example computer device 300, in combination with the operating system 400, to operate as a device performing a particular function. For example, the application 410 may cooperate with the operating system 400 to adapt a suitable embodiment of the example computer device 300 to operate as the computing device 240 (FIG. 2) of the user device 110, relying party device 120 or healthcare communication device 155 (FIG. 1), the application server 130, the healthcare data server 150 and the certificate authority server 160. While a single application 410 is illustrated in FIG. 3, in operation the memory 320 may include more than one application 410 and different applications 410 may perform different operations. For example, in at least some embodiments in which the computer device 300 is functioning as the user device 110, the relying party device 120 and/or the healthcare communication device 155, the applications 410 may include a health passport application. For example, where the health passport application is executing on the user device 110, the user may use the health passport application to request a certificate such as for example an immunisation certificate.

Embodiments of operations performed when the health passport application is executing on the user device 110, the relying party device 120 and/or the healthcare communication device 155 will now be described.

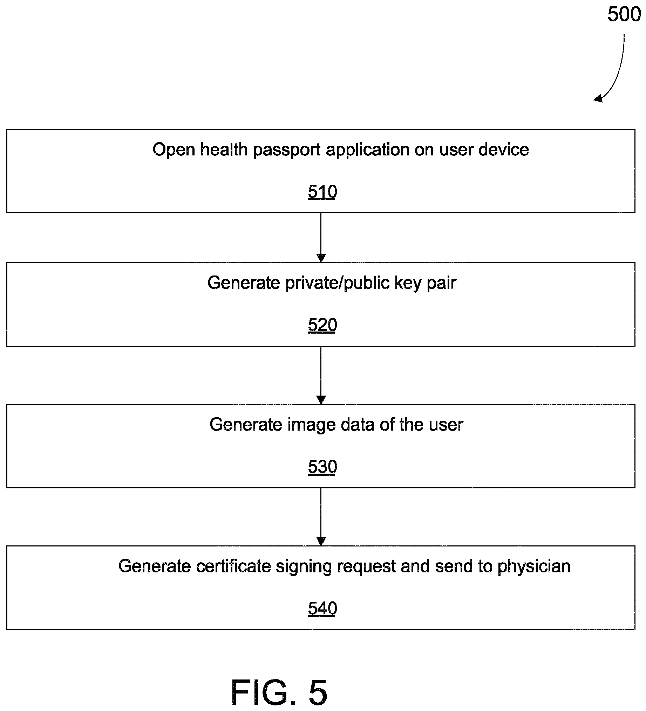

FIG. 5 is a flowchart showing operations performed according to an embodiment. The operations may be included in a method 500 which may be performed by the user device 110. For example, computer-executable instructions stored in memory of the user device 110 may, when executed by a processor user device 120, configure the user device 110 to perform the method 500 or a portion thereof.

The method 500 begins when the user opens or launches the health passport application on the user device 110 (step 510). In this embodiment, the user may wish to open or launch the health passport application prior to visiting their physician or at the time they are visiting their physician.

The health passport application may be opened or launched via input such as touch input made on a display screen of the user device 110. In response, the user device 110 opens up a graphical user interface associated with the health passport application.

Once launched, the user initiates a certificate signing request and in response the health passport application causes a private key and a public key pair to be generated (step 520). In an embodiment, the private key and public key pair may be generated by the health passport application. In another embodiment, the health passport application may not generate the private key and public key pair itself, but rather may access a hardware module configured to generate cryptographic keys such as for example Secure Enclave, which is a hardware-based key manager isolated from the processor of the user device 110. Use of the private key and public key pair will be described in more detail below.

Data in the form of image data is generated of the user (step 530). In this embodiment, the image data includes an image of the user (non-obscured, non-obfuscated or non-deformed) and a corresponding obscured, obfuscated or deformed image of the user. The image of the user may be obtained from an image library associated with the user device 110 or may be obtained using the image capture module 220 of the user device 110. The image may be for example a picture or a portrait of a face of the user.

Once the image of the user has been obtained, a corresponding obscured, obfuscated or deformed image of the user may be generated. In this embodiment, the corresponding obscured image of the user may be generated using one or more image obscuring, obfuscating or deforming methods and applying the one or more image obscuring, obfuscating or deforming methods to the image of the user. Exemplary obscuring, obfuscating or deforming methods include one or more of pixilation, blurring, obscuring, overlaying, applying a distortion effect via a filter, etc.

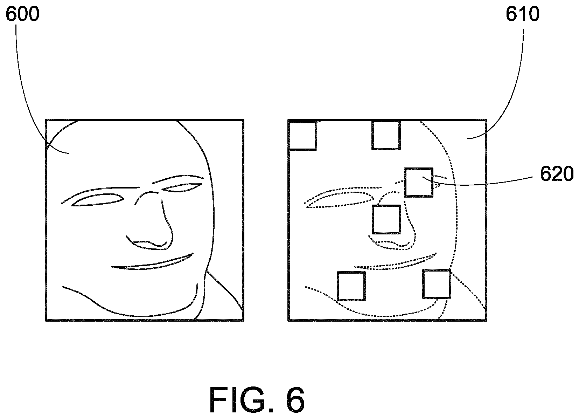

FIG. 6 shows an example image 600 and corresponding obscured image 610 of a user. In this example, the corresponding obscured image 610 is a blurred version of the image 600 and includes overlaid features 620.

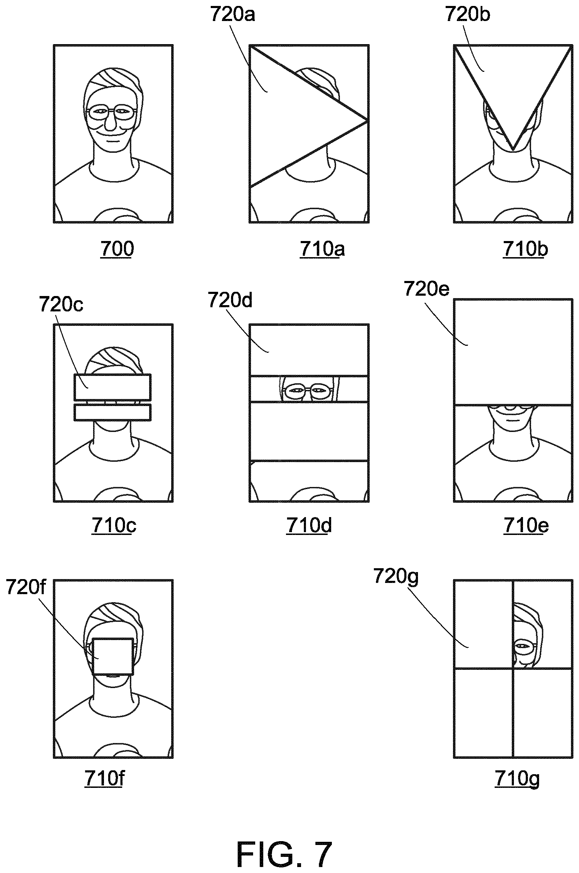

FIG. 7 shows an example image 700 and corresponding obscured images 710a, 710b, 710c, 710d, 710e, 710f and 710g of a user. In these examples, the corresponding obscured images 710a, 710b, 710c, 710d, 710e, 710f and 710g are the same as image 700 with the addition of overlaid features 720a, 720b, 720c, 720d, 720e, 720f and 720g, respectively.

Once the image data of the user has been generated, a hash of the corresponding obscured image of the user may be generated. In this embodiment, the hash of the corresponding obscured image of the user is signed using the private key generated during step 510. As mentioned, in another embodiment the health passport application may access a Secure Enclave or similar type or processor to perform the signature with the private key.

Once the image data of the user has been obtained, the certificate signing request is sent to the healthcare communication device 155 associated with the physician (step 540). In this embodiment, the certificate signing request may be for example a request for an immunisation certificate, a request for a vaccination certificate, etc. Put another way, the certificate signing request may be a request for an updated health status of the user based on an upcoming or currently occurring visit to the physician.

The request may be sent to the healthcare communication device 155 via the network 140. In this embodiment, the certificate signing request includes the signed hash of the corresponding obscured photo, the public key and the corresponding obscured photo. The certificate signing request also includes information related to the health status of the user. For example, the certificate signing request may identify one or more immunisations or vaccinations. Where an immunisation is identified, the certificate signing request may be a request for an immunisation certificate. Similarly, where a vaccination is identified, the certificate signing request may be a request for a vaccination certificate.

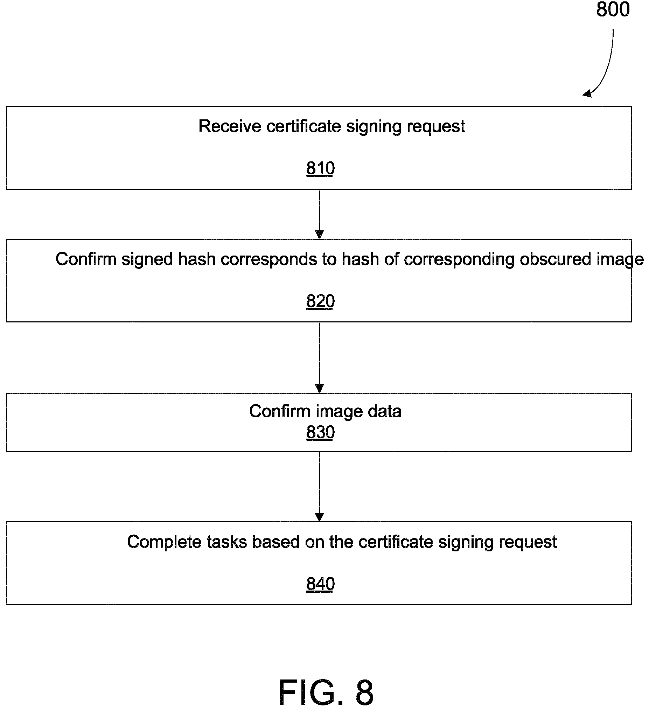

The healthcare communication device 155 receives the certificate signing request and in response performs operations. FIG. 8 is a flowchart showing operations performed by the healthcare communication device 155 according to an embodiment. The operations may be included in a method 800 which may be performed by the healthcare communication device 155. For example, computer-executable instructions stored in memory of the healthcare communication device 155 may, when executed by a processor of the healthcare communication device 155, configure the healthcare communication device 155 to perform the method 800 or a portion thereof.

The method begins when the certificate signing request is received from the user device 110 (step 810). As mentioned, the certificate signing request includes the signed hash of the corresponding obscured photo, the public key and the corresponding obscured photo. The certificate signing request also includes information related to the health status of the user. The signed hash is compared to the hash of the corresponding obscured image, and a verification of the signature is performed by using the received public key to confirm authenticity of the corresponding obscured image (step 820).

The image data is confirmed to be image data of the user (step 830). In this embodiment, the image of the user is displayed by the user on the user device 110 and this displayed image is shown to the physician to ensure it is indeed an image of the user. The corresponding obscured image is displayed on the healthcare communication device 155 and the physician confirms that the corresponding obscured image indeed corresponds to the non-obscured image of the user.

Once the hash and image data of the user are confirmed, it is determined that the user is indeed the user shown in the images and as such the physician completes one or more tasks based on the certificate signing request received from the user device 110 (step 840). For example, where the certificate signing request is a request for an immunisation certificate, the physician may obtain a sample such as a blood sample and send the sample out for analysis. In this example, a third party may be relied upon to analyze the sample and the results of the analysis may be communicated to the healthcare data server 150. Where the certificate signing request is a request for a vaccination certificate, the physician may inject the user with one or more vaccines.

Once the physician has completed the one or more tasks based on the certificate signing request, the physician may use the healthcare communication device 155 to provide information to the healthcare data server 150. For example, where the certificate signing request is a request for a vaccination certificate, the physician may provide information indicating that the user has been vaccinated.

Once information has been provided by the physician, the healthcare data server 150 generates a unique certificate identification number for the certificate signing request and updates a corresponding medical record of the user to update the health status of the user. For example, where the certificate signing request is a request for a vaccination, the medical record of the user is updated to indicate the type of vaccinations the user has received from the physician. The medical record may also be updated to include the date of the vaccination and/or an expiration date for the vaccination. The medical record is also updated to include the certificate identification number.

In some embodiments, such as for example where a third party is relied upon to analyze a sample obtained from the user, the medical record of the user may be updated before receiving the results from the third party. As such, the medical record may be updated to indicate that the user is awaiting results and the medical record may be updated once the test results are received.

Once the medical record has been updated, the unique certificate identification number is communicated to the physician and this may be done via the healthcare communication device 155. The healthcare communication device 155 may in-turn communicate the unique certificate identification number to the user device 110 where it is stored in memory. In at least some embodiments, the unique certificate identification number may only be provided to the user device 110 when the signed hash is validated.

The certificate signing request is sent by the healthcare communication device 155 to the certificate authority server 160. The certificate signing request includes the certificate identification number, the public key, the hash of the associated obscured image and information regarding the health status of the user. For example, where the certificate signing request is a request for a vaccination certificate, the certificate signing request includes information indicating that the user has been vaccinated for a particular disease. The certificate signing request may include the date the user has been vaccinated and may include an expiration date for the vaccination. As another example, where the certificate signing request is a request for an immunisation certificate, the certificate signing request may include the immunisation status of the user (immune/not immune), a start date of immunisation status, an end date of immunisation status, and one or more diseases for which the user has been determined to be immune.

Once received, the certificate authority server 160 analyzes the certificate signing request to generate or otherwise sign the requested certificate. Specifically, the certificate authority server 160 performs a check to determine that the RA is authorized and authentic and that the attributes are valid. For example, the certificate authority server 160 may determine, based on information included with the certificate signing request, that the RA is a trusted partner. The certificate authority server 160 may also ensure compliance with the public key infrastructure (PKI) policy, etc. When it is determined that the RA is authorized and authentic, it is assumed that the health status data is valid and as such the certificate authority server 160 generates or otherwise signs the requested certificate. It will be appreciated that the health status data is assumed to be valid in that the health status data represented an accepted and allowable set of health related attributes and may also mean that the values of any such attributes fall within an accepted and allowable range. The certificate includes the certificate identification number, the public key, the hash of the associated obscured image and information regarding the health status of the user. For example, where the certificate signing request is a request for an immunisation certificate, the certificate includes the immunisation status of the user (immune/not immune), a start date of immunisation status, an end date of immunisation status, and one or more diseases for which the user has been determined to be immune. As another example, where the requested certificate is a request for a vaccination certificate, the certificate includes information indicating that the user has been vaccinated for a particular disease. The certificate signing request may also include the date the user has been vaccinated and may include an expiration date for the vaccination.

Once generated, the certificate authority server 160 sends the certificate to the application server 130. The certificate authority server 160 does not store any identification information of the user. Rather, the certificate authority server 160 confirms the authenticity of the RA, generates the requested certificate and sends the certificate to the application server 130.

The application server 130 receives the certificate and stores the certificate in memory. The application server 130 sends a notification to the healthcare communication device 155 that causes the healthcare communication device 155 to display a message indicating that the certificate is available. The physician may then send a notification from the healthcare communication device 155 to the user device 110 causing the user device 110 to display a message indicating that the certificate is available. In another embodiment, the certificate may automatically be obtained by the user device 110 such as for example through the health passport application and a message may be displayed to the user indicating that the certificate has been retrieved.

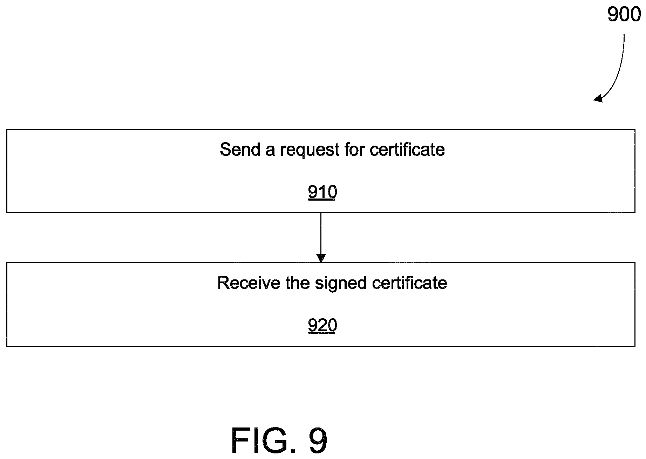

The user may now request the certificate from the application server 130. FIG. 9 is a flowchart showing operations performed by user device 110 in obtaining the certificate according to an embodiment. The operations may be included in a method 900 which may be performed by user device 110. For example, computer-executable instructions stored in memory of the user device 110 may, when executed by a processor of the user device 110, configure the user device 110 to perform the method 900 or a portion thereof.

The user device 110 sends, to the application server 130, a request for the certificate (step 910). In this embodiment, the request may include the certificate identification number and this may be signed or encrypted with the private key. The request for the certificate may include either the public key of the user or a hash of the public key of the user. For example, in another embodiment, the request may be based on the public key and a lookup may be performed to determine the appropriate certificates using the public key or a hash of the public key, where the public key and/or hash of the public key is provided to the application server 130 by the user device 110. To ensure the user device 110 is in possession of the private key, a string, timestamp or other identifying code may be signed with the private key and sent.

The application server 130 receives the request. The application server 130 obtains the requested certificate, using the certificate identification number, and performs a check to determine that elements of the request provided by the user device 110 that were signed or encrypted using the private key are valid for the particular public key. When it is determined that the elements of the request were correctly signed or encrypted, the certificate is sent to the user device 110.

The user device 110 receives the certificate from the application server 130 (step 920). As mentioned, the certificate includes the certificate identification number, the public key, the hash of the associated obscured image and information regarding the health status of the user. For example, where the certificate signing request is a request for an immunisation certificate, the certificate includes the immunisation status of the user (immune/not immune), a start date of immunisation status, an end date of immunisation status, and one or more diseases for which the user has been determined to be immune. As another example, where the requested certificate is a request for a vaccination certificate, the certificate includes information indicating that the user has been vaccinated for a particular disease. The certificate signing request may also include the date the user has been vaccinated and may include an expiration date for the vaccination. The certificate may be stored in memory of the user device 110.

When a user wishes to enter a public event or building, the relying party may wish to check if the user is healthy. For example, the relying party may wish to confirm that the user does not have a particular disease. The certificate may be used by the user to gain entry into the public event.

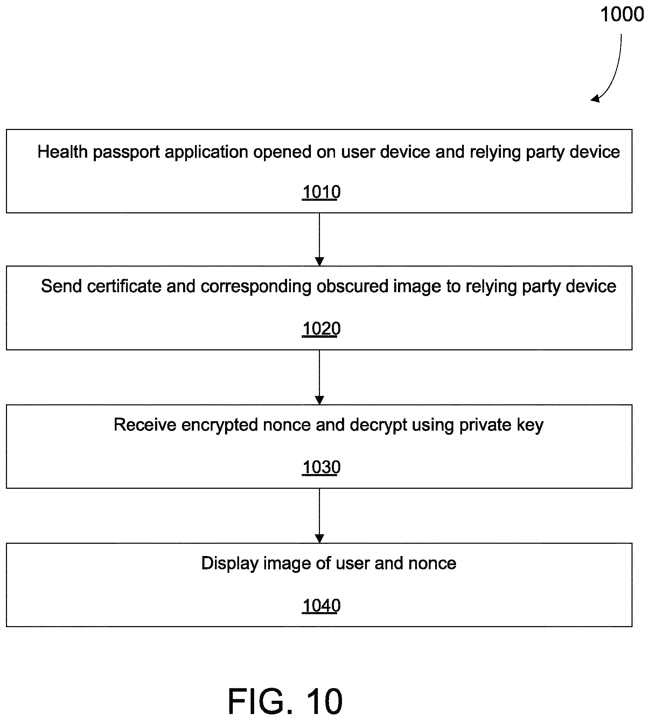

FIG. 10 is a flowchart showing operations performed by the user device 110 in providing a signed certificate to a relying party device 120 according to an embodiment. The operations may be included in a method 1000 which may be performed by the user device 110. For example, computer-executable instructions stored in memory of the user device 110 may, when executed by a processor of the user device 110, configure the user device 110 to perform the method 1000 or a portion thereof.

The relying party may verbally request that the user prove their health status prior to allowing them to entry the public event or building. As such, the method 1000 begins when the health passport application is opened on the user device 110 and the relying party device 120 (step 1010). In this embodiment, the user device 110 and the relying party device 120 are paired using Bluetooth and/or peer-to-peer discovery. It will be appreciated that in other embodiments the user device 110 and the relying party device 120 may be paired using other techniques such as through the use of QR codes

Once paired, the user device 110 sends the signed certificate and the corresponding obscured image of the user to the relying party device 120 (step 1020). Prior to sending the certificate and the corresponding obscured image of the user, a prompt may be displayed on the user device 110 asking the user to confirm that they would like to share this information with the relying party.

The corresponding obscured image of the user may be encrypted using the private key generated during method 500. The current time may also be sent to the relying party device 120 and may be signed using the private key.

In response to receiving the certificate, the corresponding obscured image of the user, and the current time, the relying party device 120 may check that the current time received from the user device 110 is indeed the current time. The relying party device 120 then determines that the certificate is valid in that the certificate has been correctly signed by the certificate authority and has not expired. Once it is determined that the current time is indeed the current time and that the certificate is valid, the relying party device 120 may generate a nonce. The nonce may be encrypted using the public key and may be sent to the user device 110.

The user device 110 receives the encrypted nonce and decrypts the nonce using the private key (of the public key and private key pair) (step 1030).

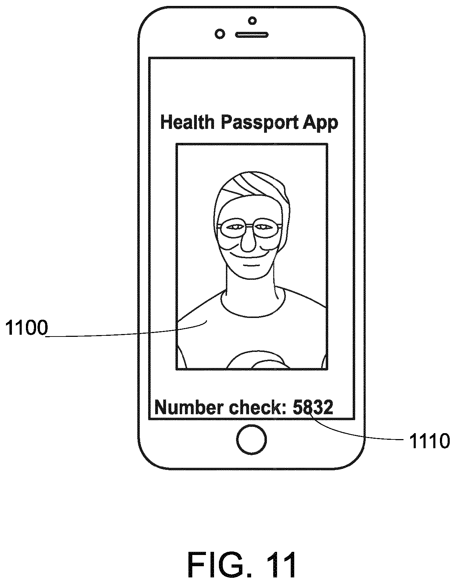

Once the nonce is decrypted, the user device 110 displays the image of the user along with the nonce (step 1040). In this embodiment, the nonce is displayed on the user device 110 below the image of the user. An example is shown in FIG. 11. As can be seen, an image 1100 of the user is displayed as well as nonce 1110. In another example, the nonce may be displayed such that it overlaps with the image of the user. In another example, the nonce may be displayed in a particular location with respect to the image of the user and/or in a particular location on the display screen of the user device 110. For example, the nonce may be displayed in the bottom right corner. In another example, the nonce may be displayed at a random location on the display screen of the user device 110.

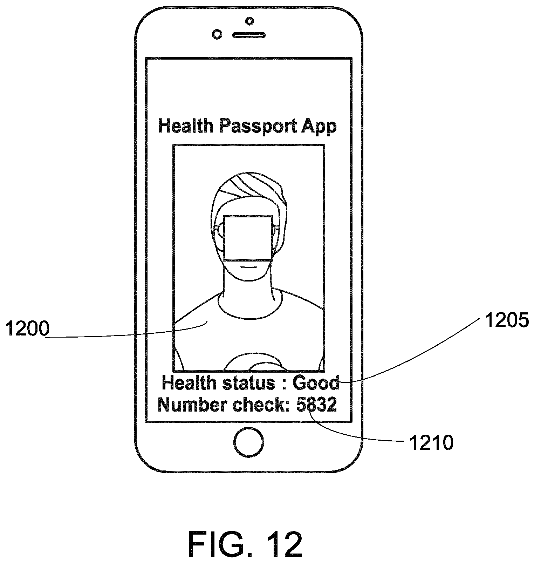

After sending the nonce to the user device 110, the relying party device 120 decrypts the corresponding obscured image of the user. The corresponding obscured image of the user is then displayed on the relying party device 120 along with the information indicating the heath status of the user. The nonce is also displayed on the relying party device 120 and may be, for example, positioned below the corresponding obscured image of the user. An example is shown in FIG. 12. As can be seen, a corresponding obscured image 1200 of the user is displayed as well as the information indicating the health status of the user 1205 and the nonce 1210. The nonce may be, for example, displayed on a display screen of the relying party device 120 at the same location as it is displayed on the display screen of the user device 110. The relying party may compare the nonce displayed on the user device 110 and the nonce displayed on the relying party device 120 to determine that they are the same value and are positioned in the same location. If the nonces are not displayed in the same location, the relying party may determine that the user is not who they say they are and may refuse the user entry.

The relying party then checks the image of the user (displayed on the user device 110) to ensure that the face of the user presenting the user device 110 corresponds to that of the image that is presented on the user device 110. The relying party then checks the corresponding obscured image of the user to ensure that the corresponding obscured image of the user, provided on the relying party's device, indeed corresponds to the non-obscured image of the user as presented on the user device 110. The relying party then checks that the nonce displayed on the user device 110 matches the nonce displayed on the relying party device 120. The relying party then checks the information indicating the health status of the user to ensure that the user is allowed to enter. For example, the relying party checks to see that the user has been vaccinated or is immune to a particular disease and thus is granted entry.

The nonce may include or may be a code such as for example a six (6) digit code. The nonce may be overlaid on top of the obscured image displayed on the relying party device and the nonce may be overlaid on top of the non-obscured image displayed on the user device. In one or more embodiments, each time the obscured image or non-obscured image are displayed, the nonce may be overlaid in a random location. In another embodiment, the nonce may be displayed on the relying party device in a manner such that the location may be controlled by the relying party. The nonce displayed on the user device may be adjusted based on the actions performed by the relying party. For example, the relying party may select the nonce via touch input on the display screen of the relying party device. The relying party may perform a gesture by moving their finger in a particular pattern and in response the location of the nonce may be updated accordingly. The nonce on the user device may follow the movement of the nonce to ensure that the user device and thus the user are legitimate. In another embodiment, both the nonce and the image may be moved via touch input. In another embodiment, the nonce and/or the image may be moved automatically on the relying party device and the user device. The movement may be, for example, random movement. In another embodiment, the relying party device may be moved and in response the nonce and/or the image may be moved. For example, movement of the relying party device may be detected via one or more sensors of the relying party device. When it is determined that the relying party device is moving, the location of the nonce and/or the image may be updated. It will be appreciated that rather than monitoring input and/or movement of the relying party device, the user device may be monitored and the location of the nonce and/or image may be updated accordingly on the user device and/or relying party device.

In this manner, it is difficult for a user to imitate or pretend to have a valid health passport as it is difficult for the user to overlap a photo on top of an image displayed by a valid health passport application on a user device. Put another way, a user cannot simply pretend to have a valid health passport by overlaying their image on someone else's valid health passport.

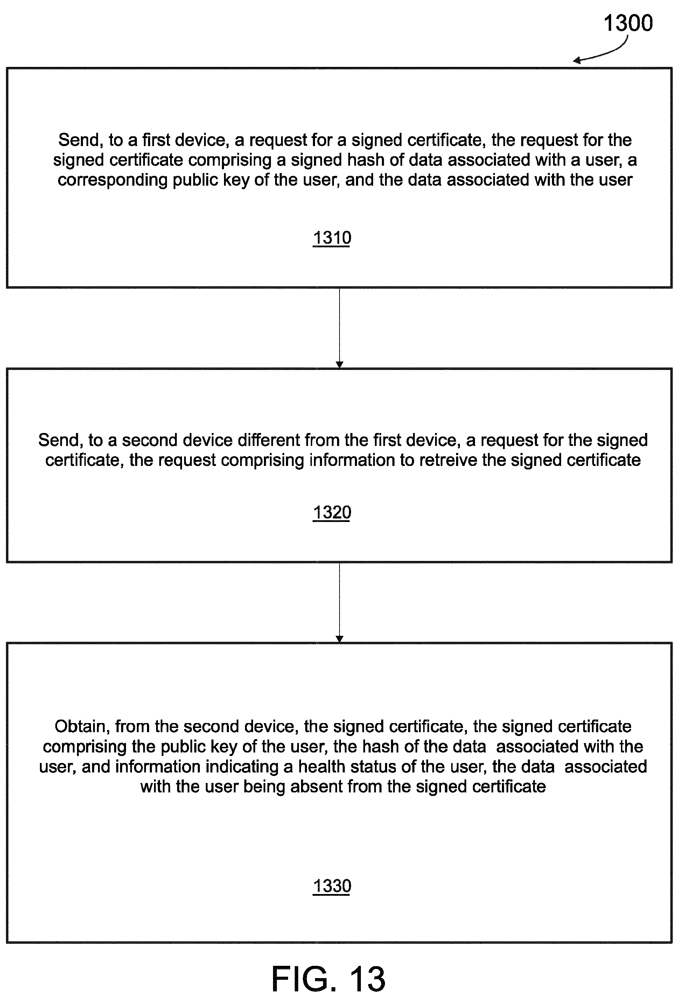

FIG. 13 is a flowchart showing operations for obtaining a signed certificate according to another embodiment. The operations may be included in a method 1300 which may be performed by the user device 110. For example, computer-executable instructions stored in memory of the user device 110 may, when executed by a processor user device 120, configure the user device 110 to perform the method 1300 or a portion thereof.

The user device 110 sends, to a first device, a request for a signed certificate, the request for the signed certificate comprising a signed hash of data associated with a user, a corresponding public key of the user, and the data associated with the user (step 1310). In this embodiment, the first device may include the healthcare communication device 155 and/or the healthcare data server 150 and the first device may be caused to perform one or more steps to obtain or otherwise generate a certificate in manners similar to that described herein. The data associated with the user may correspond to partial personally identifiable information. The partial personally identifiable information may correspond to information available on an identity document of the user such as a personal identity card.

The user device 110 sends, to a second device different than the first device, a request for the signed certificate, the request comprising information to retrieve the signed certificate (step 1320). The second device may include the certificate authority server 160 and the second device may be caused to perform one or more steps to obtain or otherwise generate a signed certificate in manners similar to that described herein.

The user device 110 obtains, from the second device, the signed certificate, the signed certificate comprising the public key of the user, the hash of the data associated with the user, and information indicating a health status of the user, the data associated with the user being absent from the signed certificate (step 1330). As mentioned, the second device may include the certificate authority server 160 which may be caused to perform one or more steps to obtain or otherwise generate the signed certificate.

In embodiments described herein, limited or non-identifying personal information about the user is not shared with the relying party or the relying party device. Put another way, the information shared with the relying party or the relying party device would be insufficient for a recipient to associate an identity of the user with the personal health data. Only an obscured image of the user and a signed certificate are shared with the relying party. Further, the relying party has improved confidence that the user is indeed the user associated with the certificate by generating the nonce and sending the nonce in an encrypted manner. Further, the relying party can ensure the user is indeed the user associated with the certificate by ensuring that the nonce is displayed on a display screen of the relying party device 120 at the same location as it is displayed on the display screen of the user device 110.

In one or more embodiments, additional data may be shared with the relying party device to increase the relying party's confidence that the information on the health passport corresponds to or is associated with the user presenting the user device 110. For example, biometric data such as a height, weight, eye colour, gender, age, etc. of the user may be shared and may be displayed on the relying party device. This may further help the relying party confirm that the certificate is truly associated with the user that is presenting the user device. The additional data may be shared as a range of data. For example, the additional data may indicate that the user is between 180 lbs and 200 lbs.

In one or more embodiments, the relying party may require additional identification methods to confirm that the certificate is truly associated with the user that is presenting the user device. For example, the relying party may request a personal identification card such as for example a driver's license or passport of the user. Information obtained from the personal identification card may be compared to that included with the signed certificate to further confirm that the certificate is truly associated with the user that is presenting the user device.

In one or more embodiments, multiple signed certificates may be obtained by the user. For example, a first signed certificate may be an immunisation certificate and may include the immunisation status of the user, a corresponding obscured image of the user, and a public key. A second signed certificate may include the immunisation status of the user, the full legal name of the user, and a date of birth of the user. A third signed certificate may include the immunisation status of the user, a partial name of the user (such as a first name of the user), and a partial date of birth of the user (such as the year of birth of the user). The user may select which signed certificate to provide to the relying party device. Alternatively, the relying party may request particular information regarding the user and the user device may receive a prompt requesting confirmation that the user device may share the requested information with the relying party via the relying party device. For example, the relying party may request the day of the month the user was born and the immunisation status of the user. The user may receive a prompt on the user device requesting confirmation that the user device can provide this information to the relying party device. When the user grants permission, the requested information may be provided to the relying party device.

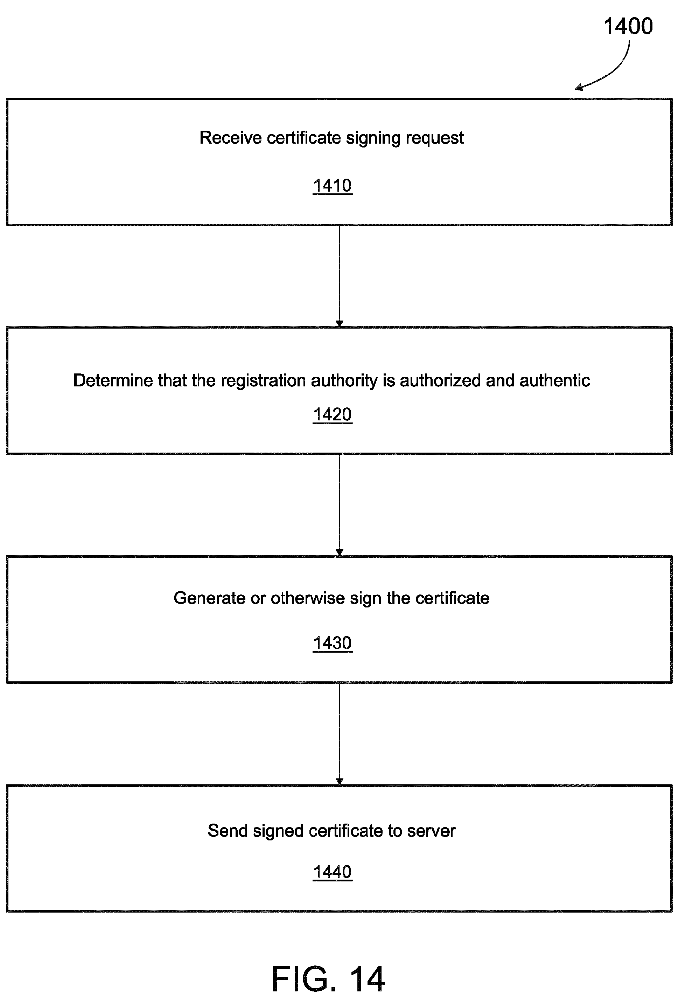

In embodiments described above, the certificate authority server 160 is described as generating or otherwise signing a requested certificate. FIG. 14 is a flowchart showing operations performed by the certificate authority server 160 in generating or otherwise signing a requested certificate according to an embodiment. The operations may be included in a method 1400 which may be performed by the certificate authority server 160. For example, computer-executable instructions stored in memory of the certificate authority server 160 may, when executed by a processor of the certificate authority server 160, configure the certificate authority server 160 to perform the method 1400 or a portion thereof.

The certificate authority server 160 receives, from the healthcare communication device or healthcare server, a certificate signing request (step 1410). In this embodiment, the certificate signing request includes the certificate identification number, the public key, the hash of the associated obscured image and information regarding the health status of the user.

The certificate authority server 160 determines that the registration authority (RA) is authorized and authentic (step 1420). In this embodiment, the certificate authority server 160 identifies and authenticates the RA based on information provided with the certificate signing request. The certificate authority server 160 may have access to a whitelist that includes trusted registration authorities and determined that the RA is indeed included in the whitelist and thus is authorized and authentic.

When it is determined that the RA is authorized and authentic, the certificate authority server 160 generates or otherwise signs the certificate (step 1430). The certificate may include the certificate identification number, the public key, the hash of the associated obscured image and information regarding the health status of the user. For example, where the certificate signing request is a request for an immunisation certificate, the certificate includes the immunisation status of the user (immune/not immune), a start date of immunisation status, an end date of immunisation status, and one or more diseases for which the user has been determined to be immune. As another example, where the requested certificate is a request for a vaccination certificate, the certificate includes information indicating that the user has been vaccinated for a particular disease. The certificate signing request may also include the date the user has been vaccinated and may include an expiration date for the vaccination.

Once generated, the certificate authority server 160 sends the certificate to the application server 130 (step 1440). The certificate authority server 160 does not store any identification information of the user. The certificate authority server 160 confirms the authenticity of the RA, generates the requested certificate and sends the certificate to the application server 130.

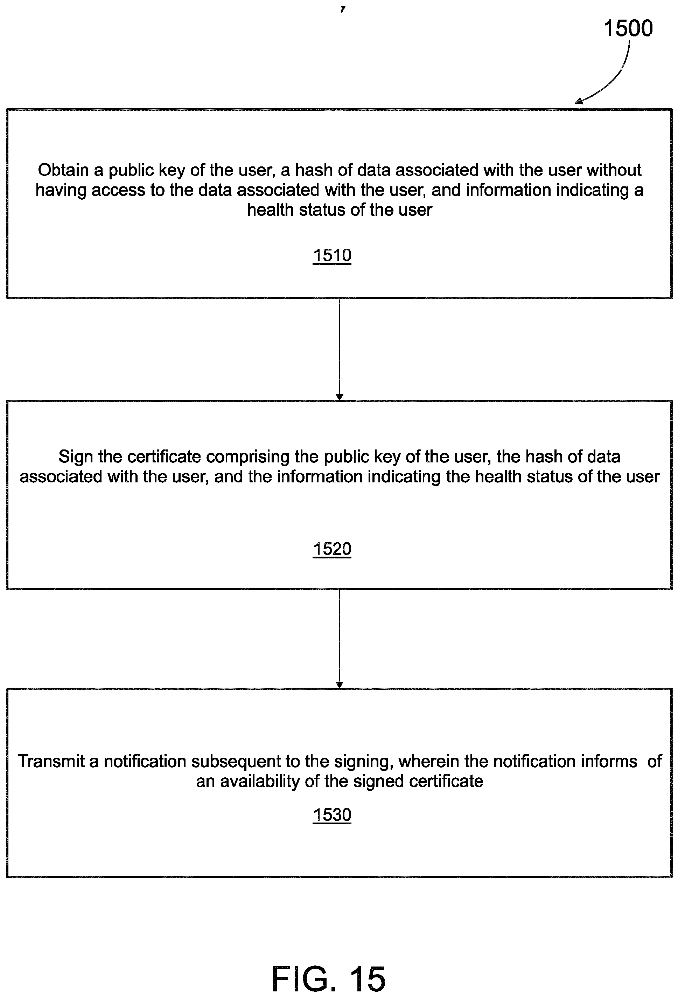

FIG. 15 is a flowchart showing operations performed by the certificate authority server 160 in generating or otherwise signing a requested certificate according to another embodiment. The operations may be included in a method 1500 which may be performed by the certificate authority server 160. For example, computer-executable instructions stored in memory of the certificate authority server 160 may, when executed by a processor of the certificate authority server 160, configure the certificate authority server 160 to perform the method 1500 or a portion thereof.

The certificate authority server 160 obtains a public key of a user, a hash of data associated with the user, without having access to the data associated with the user, and information indicating a health status of the user from a communication with a first device (step 1510). In this embodiment, the first device may be the healthcare communication device or healthcare server described herein. This step may be completed in manners similar to step 1410 described herein.

The certificate authority server 160 signs a certificate comprising the public key of the user, the hash of the data associated with the user, and the information indicating the health status of the user (step 1520). The signing of the certificate delivers a signed certificate where the data associated with the user is absent from the signed certificate. This step may be completed in manners similar to steps 1420 and 1430 described herein.

The certificate authority server 160 transmits, to the first device, a notification subsequent to the signing, wherein the notification informs of an availability of the signed certificate (step 1530). In response, the first device may retrieve the signed certificate in manners similar to that described herein.

In another embodiment, a user may wish to enter a public event or building that requires a ticket. In this embodiment, the relying party may wish to check if the user has a valid ticket and that the user is healthy. For example, prior to permitting entry into an event, the relying party may wish to confirm that the user has a ticket for the event and that the user has been vaccinated for a particular disease.

In this embodiment, a scannable code is generated that includes the event ticket and data associated with a health certificate of the user. The data may include, for example, partial personally identifiable information of a user, information regarding the health status of a user, and/or a single use code that may be used to obtain the health certificate of the user. As will be described in more detail below, the scannable code is configured to be scanned by a relying party device and to cause the relying party device to display at least a notification indicating a ticket status and information associated with the user. The information associated with the user may include, for example, the partial personally identifiable information of a user, the heath status of the user and/or an obscured image of the user.

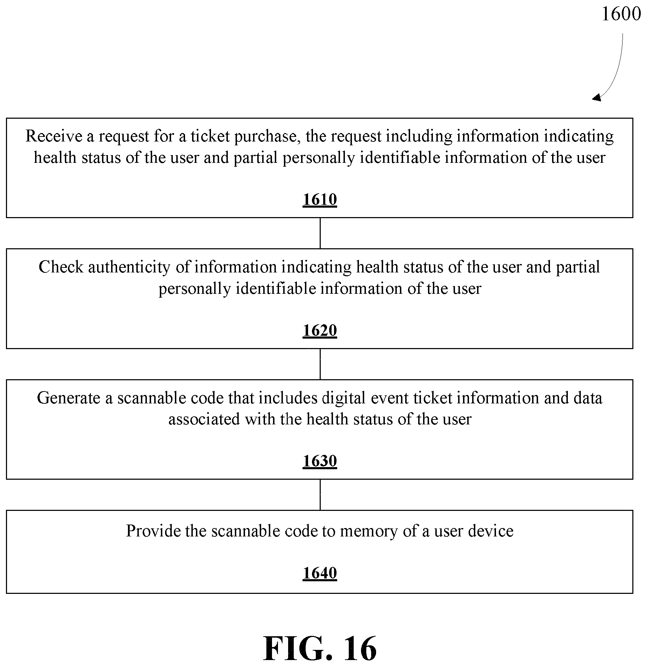

FIG. 16 is a flowchart showing operations performed in generating a scannable code that includes a digital event ticket and data associated with a health certificate of the user. The operations may be included in a method 1600 which may be performed by a ticketing server associated with a ticket service. For example, computer-executable instructions stored in memory of the ticketing server may, when executed by a processor of the ticketing server, configure the ticketing server to perform the method 1600 or a portion thereof.

The ticketing server receives a request for a ticket purchase, the request including information indicating health status of the user and partial personally identifiable information of the user (step 1610). In this embodiment, the request may be received from the user device 110. Specifically, the user of the user device 110 may submit a request for the ticket purchase using, for example, a ticket mobile application executing on the user device 110 or a ticket mobile website accessed through a web browser executing on the user device 110. The ticket mobile application or ticket mobile website is associated with the ticketing server.

In this embodiment, the information indicating health status of the user and partial personally identifiable information of the user may be included with a signed certificate. For example, the user may obtain a signed certificate according to method 1300 described herein and as such the signed certificate may include a public key of the user, hash of the data associated with the user, and information indicating a health status of the user. The information indicating health status of the user may include partial personally identifiable information of the user such as for example their name, initials, age, height, weight, eye colour, hair colour, birthdate, etc. The signed certificate may be provided by the user device 110 to the ticketing server.

The ticketing server checks the authenticity of the health status of the user and partial personally identifiable information of the user (step 1620). In this embodiment, the server checks the authenticity of the signed certificate and that the health status of the user will be valid on the day of the event. The ticketing server compares the partial personally identifiable information to identity information of the user. For example, credit card information received with the request for the ticket purchase may include identity information of the user and this may be compared to the partial personally identifiable information to ensure they match.

A scannable code is generated which includes digital event ticket information and data associated with the health status of the user (step 1630). As mentioned, in this embodiment the data associated with the health status of the user includes the information indicating the health status of the user and the partial personally identifiable information.

In this embodiment, the scannable code is in the form of a quick response (QR) code. The QR code is generated such that, when scanned or read by a device such as the relying party device 120, the QR code causes the relying party device to display a notification indicating ticket status and information associated with the user. In this embodiment, the information associated with the user includes the health status of the user and the partial personally identifiable information.

The scannable code is provided to memory of the user device 110 (step 1640). The scannable code may be provided, for example, to the user device 110 in the form of a digital event ticket. The user device 110 stores the scannable code in memory.

When the user wishes to enter the event for which the ticket was purchased, the user presents the scannable code to the relying party. For example, the scannable code may be displayed on a display screen of the user device 110. The relying party scans the scannable code using the relying party device 120. The relying party device 120 determines a ticket status of the scanned event ticket, that is, whether or not the ticket is valid for the event.

To determine ticket status, in this embodiment, the relying party device 120 may send a signal including information regarding the digital event ticket to the ticketing server. Responsive to receiving the signal, the ticketing server may determine whether or not the ticket is valid and in turn may send a signal including the ticket status to the relying party device 120. The ticket status may indicate whether or not the scanned ticket is a valid ticket for the event.

Responsive to determining that the ticket is a valid ticket for the event, the relying party device 120 displays a notification indicating ticket status and information associated with the user. Again, in this embodiment, the information associated with the user includes information indicating the health status of the user and the partial personally identifiable information of the user.

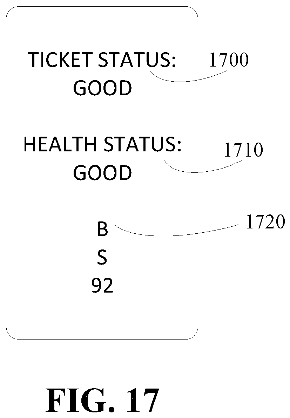

An example is shown in FIG. 17. As can be seen, the relying party device 120 displays the ticket status 1700, health status 1710 and partial personally identifiable information of the user 1720. In this example, the ticket status 1700 indicates that the event ticket is good and the health status 1710 indicates that the user's health is good. The partial personally identifiable information 1720 includes the initials of the user ("B" for Bob and "S" for Smith) and the last two digits of the user's birth year ("92" for "1992"). The relying party may use this information to permit the user to enter the event.

In another embodiment, the data associated with the health status of the user may only include the partial personally identifiable information of the user. In this example, the ticketing server may generate a scannable code that includes digital ticket information and the partial personally identifiable information. The scannable code may be in the form of a QR code. The QR code may be generated such that, when scanned or read by a device such as a relying party device 120, the QR code causes the relying party device to display a notification indicating ticket status and information associated with the user. The information associated with the user includes the partial personally identifiable information.

When the user wishes to enter the event for which the ticket was purchased, the user presents the scannable code to the relying party. For example, the scannable code may be displayed on a display screen of the user device 110. The relying party scans the scannable code using the relying party device 120. The relying party device 120 determines a ticket status of the scanned event ticket, that is, whether or not the ticket is valid for the event. Responsive to determining that the ticket is a valid ticket for the event, the relying party device 120 displays a notification indicating ticket status and the information associated with the user (which in this embodiment is the partial personally identifiable information).

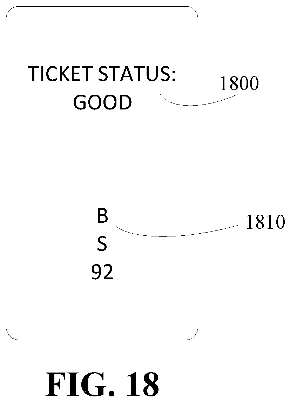

An example is shown in FIG. 18. As can be seen, the relying party device 120 displays the ticket status 1800 and the partial personally identifiable information 1810 of the user. In this example, the ticket status 1800 indicates that the event ticket is good. The partial personally identifiable information 1810 includes the initials of the user ("B" for Bob and "S" for Smith) and the last two digits of the user's birth year ("92" for "1992").

Responsive to the ticket status indicating that the event ticket is good, the relying party may request health status information of the user. The user may provide health status information using the signed certificate similar to embodiments described above. For example, similar to method 1000 described herein, the health passport application may be opened on the user device 110 and the relying party device 120. In this embodiment, the user device 110 and the relying party device 120 may be paired using Bluetooth and/or peer-to-peer discovery. It will be appreciated that in other embodiments the user device 110 and the relying party device 120 may be paired using other techniques such as through the use of QR codes.

Once paired, the user device 110 may send the signed certificate and, optionally, a corresponding obscured image of the user to the relying party device 120. Prior to sending the certificate and the corresponding obscured image of the user, a prompt may be displayed on the user device 110 asking the user to confirm that they would like to share this information with the relying party.

Responsive to receiving the signed certificate, the relying party/relying party device 120 may check the authenticity of the signed certificate. For example, partial personally identifiable information included with the certificate may be compared to the partial personally identifiable information obtained when scanning the QR code to ensure they match. The health status of the user may then be checked by the relying party. In one embodiment of the disclosure, the health status of the user may be displayed on the relying party screen as shown in FIG. 17.

In one or more embodiments, the relying party may request additional information of the user. For example, the relying party may request that the user also provide them with, for example, a personal identification card. In this manner, information obtained from the personal identification card may be compared to that displayed on the relying party device 120 to confirm that the user is legitimate. As another example, the relying party may request that the user provide the relying party device 120 with an obscured photo similar to embodiments described herein. As yet another example, a nonce may be used similar to embodiments described herein.

In another embodiment, the data associated with the digital health certificate of the user includes a single use health status code. In this embodiment, the user may select an option within the health passport application to retrieve a single use health status code. The single use health status code may be provided to the ticketing server and the scannable code may be generated to include the single use health code in manners similar to that described herein.

When the user wishes to enter the event for which the ticket was purchased, the user presents the scannable code to the relying party. For example, the scannable code may be displayed on a display screen of the user device 110. The relying party scans the scannable code using the relying party device 120. The relying party device 120 determines a ticket status of the scanned event ticket, that is, whether or not the ticket is valid for the event. To determine the health status of the user, the relying party device 120 sends a signal to the application server 130, the signal including the single use health status code. Using the single use health status code, the application server 130 retrieves information indicating the health status of the user from memory and sends a signal including the information indicating the health status of the user to the relying party device 120. The relying party device 120 may display the ticket status and health status of the user in manners similar to that described herein.

In another embodiment, the single use health status code may be used by the ticketing server to obtain health status of the user. For example, responsive to scanning the scannable code, the relying party device 120 may send a signal including information regarding the digital event ticket and the single use health status code to the ticketing server. In turn, the ticketing server may send a signal to the application server 130, the signal including the single use health status code. Using the single use health status code, the application server 130 retrieves information indicating the health status of the user and sends a signal including the information indicating the health status of the user to the ticketing server.

In another embodiment, the single use health status code may further cause the application server 130 to send a photo, such as an obscured described herein, for display on the relying party device 120 to confirm the identity of the user.

Although in embodiments the scannable code is generated by the ticketing server, the scannable code may be generated by, for example, the user device 110. For example, in another embodiment, via one or more applications executing on the user device 110, data associated with an event ticket and data associated with a digital health certificate of the user may be obtained. A scannable code may then be generated based on the obtained data associated with the event ticket and the data associated with the digital health certificate. The data associated with the digital health certificate of the user may be obtained, for example, from a healthcare data server. In another embodiment, responsive to scanning the scannable code, the relying party device may determine a date related to the health status of the user (such as the date of a health test or health assessment of the user) and may generate a notification by comparing the determined date to a reference date.

Example embodiments of the present application are not limited to any particular operating system, system architecture, mobile device architecture, server architecture, or computer programming language.

It will be understood that the applications, modules, routines, processes, threads, or other software components implementing the described method/process may be realized using standard computer programming techniques and languages. The present application is not limited to particular processors, computer languages, computer programming conventions, data structures, or other such implementation details. Those skilled in the art will recognize that the described processes may be implemented as a part of computer-executable code stored in volatile or non-volatile memory, as part of an application-specific integrated chip (ASIC), *etc.*

As noted, certain adaptations and modifications of the described embodiments can be made. Therefore, the above discussed embodiments are considered to be illustrative and not restrictive.

## Claims

1. A computer-implemented method (1600) performed by a ticketing server associated with a ticketing service, the method (1600) comprising:
receiving (1610), from a user device (110, 200) associated with a user, a request for an event ticket, the request including information indicating health status of the user and partial personally identifiable information from the user;
obtaining data associated with the event ticket and data associated with a digital health certificate of the user;
generating (1630) a scannable code that includes the data associated with the event ticket and the data associated with the digital health certificate of the user, wherein the data associated with the digital health certificate includes a single use health status code, the scannable code configured to be scanned by a relying party device (120, 200) and causing the relying party device to obtain the health status of the user based on the single use health status code and to display at least a notification indicating a ticket status (1700) and information associated with the user; and
providing (1640) the scannable code to memory of the user device (110, 200);
wherein the data associated with the digital health certificate includes information indicating a health status (1710) of the user and the partial personally identifiable information from the user;
wherein the scannable code, when scanned by the relying party device, causes the relying party device to:
send, to the ticketing server associated with the ticketing service, a signal requesting ticket status;
receive, from the ticketing server associated with the ticketing service, a signal indicating the ticket status;
obtain, from the ticketing server or an application server, the health status information of the user based on the single use health status code; and
display, on a display screen of the relying party device (120), the ticket status, the health status of the user and the partial personally identifiable information (1720, 1810) of the user.

2. The computer-implemented method (1600) of claim 1, wherein the method (1600) further comprises checking (1620) the authenticity of the health status of the user and the partial personally identifiable information of the user.

3. The computer-implemented method (1600) of claim 2, wherein the information indicating health status of the user and partial personally identifiable information of the user may be included with a signed certificate, and wherein the ticketing server checks the authenticity of the signed certificate and that the health status of the user will be valid on the day of the event.

4. The computer-implemented method (1600) of claim 3, wherein the signed certificate includes a public key of the user, hash of the data associated with the user, and information indicating a health status of the user, wherein the data associated with the user corresponds to the partial personally identifiable information.

5. The computer-implemented method (1600) of claim 4, wherein the partial personally identifiable information of the user includes name, initials, age, height, weight, eye colour, hair colour or birthdate of the user.

6. The computer-implemented method (1600) of claim 1, wherein the single use health status code causes the application server to send a photo for display on the relying party device to confirm the identity of the user.

7. The computer-implemented method (1600) of claim 6, wherein the photo is obscured.

8. The computer-implemented method (1600) of claim 1, wherein the health status of the user includes at least one of a vaccination record, an immunisation status, a start date of immunisation status, an end date of immunisation status, or an indication as to whether or not the user has a particular disease.

9. The computer-implemented method (1600) of claim 1, further comprising:
receiving, at the relying party device, information indicating health status of the user, the information indicating health status of the user provided by the user device (110).

10. The computer-implemented method (1600) of any preceding claim, wherein the scannable code is a quick response, QR, code.

11. A system (100) comprising:
at least one processor (310); and
a memory (320) coupled to the at least one processor (310) and storing instructions that, when executed by the at least one processor, cause the system to carry out the method (1600) of any preceding claim.

12. A computer program which, when executed on at least one processor of a system, is configured to cause the system to carry out the method (1600) of any one of claims 1 to 10.

## Patentansprüche

1. Ein computerimplementiertes Verfahren (1600), das von einem Ticketvertriebsserver, der mit einem Ticketvertriebsservice assoziiert ist, vollzogen wird, wobei das Verfahren (1600) Folgendes beinhaltet:
Empfangen (1610) einer Anforderung eines Veranstaltungstickets von einem Nutzergerät (110, 200), das mit einem Nutzer assoziiert ist, wobei die Anforderung Informationen umfasst, die den Gesundheitsstatus des Nutzers und partielle personenbezogene Daten von dem Nutzer angeben;
Erhalten von Daten, die mit dem Veranstaltungsticket assoziiert sind, und von Daten, die mit einem digitalen Gesundheitszertifikat des Nutzers assoziiert sind;
Erzeugen (1630) eines scanbaren Codes, der die Daten, die mit dem Veranstaltungsticket assoziiert sind, und die Daten, die mit dem digitalen Gesundheitszertifikat des Nutzers assoziiert sind, umfasst, wobei die Daten, die mit dem digitalen Gesundheitszertifikat assoziiert sind, einen Gesundheitsstatus-Einmalcode umfassen, wobei der scanbare Code dazu konfiguriert ist, von einem Gerät (120, 200) einer vertrauenden Partei gescannt zu werden, und bewirkt, dass das Gerät der vertrauenden Partei basierend auf dem Gesundheitsstatus-Einmalcode den Gesundheitsstatus des Nutzers erhält und mindestens eine Nachricht anzeigt, die einen Ticketstatus (1700) und mit dem Nutzer assoziierte Informationen angibt; und
Bereitstellen (1640) des scanbaren Codes für einen Arbeitsspeicher des Nutzergeräts (110, 200);
wobei die Daten, die mit dem digitalen Gesundheitszertifikat assoziiert sind, Informationen, die einen Gesundheitsstatus (1710) des Nutzers angeben, und die partiellen personenbezogenen Daten des Nutzers umfassen;
wobei der scanbare Code, wenn er von dem Gerät der vertrauenden Partei gescannt wird, bewirkt, dass das Gerät der vertrauenden Partei Folgendes macht:
Senden eines Signals, das den Ticketstatus anfordert, an den Ticketvertriebsserver, der mit dem Ticketvertriebsservice assoziiert ist;
Empfangen eines Signals, das den Ticketstatus angibt, von dem Ticketvertriebsserver, der mit dem Ticketvertriebsservice assoziiert ist;
Erhalten der Gesundheitsstatusinformationen des Nutzers von dem Ticketvertriebsserver oder einem Anwendungsserver, basierend auf dem Gesundheitsstatus-Einmalcode; und
Anzeigen des Ticketstatus, des Gesundheitsstatus des Nutzers und der partiellen personenbezogenen Daten (1720, 1810) des Nutzers auf einem Anzeigebildschirm des Geräts (120) der vertrauenden Partei.

2. Computerimplementiertes Verfahren (1600) gemäß Anspruch 1, wobei das Verfahren (1600) ferner das Prüfen (1620) der Authentizität des Gesundheitsstatus des Nutzers und der partiellen personenbezogenen Daten des Nutzers beinhaltet.

3. Computerimplementiertes Verfahren (1600) gemäß Anspruch 2, wobei die Informationen, die den Gesundheitsstatus des Nutzers und partielle personenbezogene Daten des Nutzers angeben, in einem signierten Zertifikat umfasst sein können und wobei der Ticketvertriebsserver die Authentizität des signierten Zertifikats prüft und dass der Gesundheitsstatus des Nutzers am Tag der Veranstaltung gültig sein wird.

4. Computerimplementiertes Verfahren (1600) gemäß Anspruch 3, wobei das signierte Zertifikat einen öffentlichen Schlüssel des Nutzers, einen Hash der Daten, die mit dem Nutzer assoziiert sind, und Informationen, die einen Gesundheitsstatus des Nutzers angeben, umfasst, wobei die Daten, die mit dem Nutzer assoziiert sind, den partiellen personenbezogenen Daten entsprechen.

5. Computerimplementiertes Verfahren (1600) gemäß Anspruch 4, wobei die partiellen personenbezogenen Daten des Nutzers den Namen, die Initialen, das Alter, die Körpergröße, das Gewicht, die Augenfarbe, die Haarfarbe oder das Geburtsdatum des Nutzers umfassen.

6. Computerimplementieres Verfahren (1600) gemäß Anspruch 1, wobei der Gesundheitsstatus-Einmalcode bewirkt, dass der Anwendungsserver ein Foto zum Anzeigen auf dem Gerät der vertrauenden Partei sendet, um die Identität des Nutzers zu bestätigen.

7. Computerimplementiertes Verfahren (1600) gemäß Anspruch 6, wobei das Foto verdeckt ist.

8. Computerimplementiertes Verfahren (1600) gemäß Anspruch 1, wobei der Gesundheitsstatus des Nutzers mindestens eines von einem Impfnachweis, einem Immunisierungsstatus, einem Anfangsdatum des Immunisierungsstatus, einem Ablaufdatum des Immunisierungsstatus oder einer Angabe, ob der Nutzer eine bestimmte Krankheit hat oder nicht, umfasst.

9. Computerimplementiertes Verfahren (1600) gemäß Anspruch 1, das ferner Folgendes beinhaltet:
Empfangen von Informationen, die den Gesundheitsstatus des Nutzers angeben, am Gerät der vertrauenden Partei, wobei die Informationen, die den Gesundheitsstatus des Nutzers angeben, von dem Nutzergerät (110) bereitgestellt werden.

10. Computerimplementiertes Verfahren (1600) gemäß einem der vorhergehenden Ansprüche, wobei der scanbare Code ein Quick-Response-Code, QR-Code, ist.

11. Ein System (100), das Folgendes beinhaltet:
mindestens einen Prozessor (310); und
einen Arbeitsspeicher (320), der mit dem mindestens einen Prozessor (310) gekoppelt ist und Befehle speichert, die bei Ausführung durch den mindestens einen Prozessor bewirken, dass das System das Verfahren (1600) gemäß einem der vorhergehenden Ansprüche durchführt.

12. Ein Computerprogramm, das dazu konfiguriert ist, bei Ausführung auf mindestens einem Prozessor eines Systems zu bewirken, dass das System das Verfahren (1600) gemäß einem der Ansprüche 1 bis 10 durchführt.

## Revendications

1. Un procédé mis en œuvre par ordinateur (1600) réalisé par un serveur de billetterie associé à un service de billetterie, le procédé (1600) comprenant :
la réception (1610), en provenance d'un dispositif utilisateur (110, 200) associé à un utilisateur, d'une demande pour un billet d'événement, la demande incluant des informations indiquant un état de santé de l'utilisateur et des informations personnelles identifiables partielles venant de l'utilisateur ;
l'obtention de données associées au billet d'événement et de données associées à un certificat de santé numérique de l'utilisateur ;
la génération (1630) d'un code pouvant être scanné qui inclut les données associées au billet d'événement et les données associées au certificat de santé numérique de l'utilisateur, dans lequel les données associées au certificat de santé numérique incluent un code d'état de santé à usage unique, le code pouvant être scanné étant configuré pour être scanné par un dispositif de partie utilisatrice (« *relying party* ») (120, 200) et amenant le dispositif de partie utilisatrice à obtenir l'état de santé de l'utilisateur sur la base du code d'état de santé à usage unique et à afficher au moins une notification indiquant un état de billet (1700) et des informations associées à l'utilisateur ; et
la fourniture (1640) du code pouvant être scanné à la mémoire du dispositif utilisateur (110, 200) ;
dans lequel les données associées au certificat de santé numérique incluent des informations indiquant un état de santé (1710) de l'utilisateur et les informations personnelles identifiables partielles venant de l'utilisateur ;
dans lequel le code pouvant être scanné, lorsqu'il est scanné par le dispositif de partie utilisatrice, amène le dispositif de partie utilisatrice à :
envoyer, au serveur de billetterie associé au service de billetterie, un signal demandant l'état de billet ;
recevoir, en provenance du serveur de billetterie associé au service de billetterie, un signal indiquant l'état de billet ;
obtenir, du serveur de billetterie ou d'un serveur d'application, les informations d'état de santé de l'utilisateur sur la base du code d'état de santé à usage unique ; et
afficher, sur un écran d'affichage du dispositif de partie utilisatrice (120), l'état de billet, l'état de santé de l'utilisateur et les informations personnelles identifiables partielles (1720, 1810) de l'utilisateur.

2. Le procédé mis en œuvre par ordinateur (1600) de la revendication 1, dans lequel le procédé (1600) comprend en outre la vérification (1620) de l'authenticité de l'état de santé de l'utilisateur et des informations personnelles identifiables partielles de l'utilisateur.

3. Le procédé mis en œuvre par ordinateur (1600) de la revendication 2, dans lequel les informations indiquant l'état de santé de l'utilisateur et des informations personnelles identifiables partielles de l'utilisateur peuvent être incluses dans un certificat signé, et dans lequel le serveur de billetterie vérifie l'authenticité du certificat signé et que l'état de santé de l'utilisateur sera valide le jour de l'événement.

4. Le procédé mis en œuvre par ordinateur (1600) de la revendication 3, dans lequel le certificat signé inclut une clé publique de l'utilisateur, un hash des données associées à l'utilisateur, et des informations indiquant un état de santé de l'utilisateur, dans lequel les données associées à l'utilisateur correspondent aux informations personnelles identifiables partielles.

5. Le procédé mis en œuvre par ordinateur (1600) de la revendication 4, dans lequel les informations personnelles identifiables partielles de l'utilisateur incluent le nom, les initiales, l'âge, la taille, le poids, la couleur des yeux, la couleur des cheveux ou la date de naissance de l'utilisateur.

6. Le procédé mis en œuvre par ordinateur (1600) de la revendication 1, dans lequel le code d'état de santé à usage unique amène le serveur d'application à envoyer une photo pour affichage sur le dispositif de partie utilisatrice afin de confirmer l'identité de l'utilisateur.

7. Le procédé mis en œuvre par ordinateur (1600) de la revendication 6, dans lequel la photo est occultée.

8. Le procédé mis en œuvre par ordinateur (1600) de la revendication 1, dans lequel l'état de santé de l'utilisateur inclut au moins un élément parmi un carnet de vaccination, un état d'immunisation, une date de début d'état d'immunisation, une date de fin d'état d'immunisation, ou une indication quant à savoir si l'utilisateur a ou non une maladie particulière.

9. Le procédé mis en œuvre par ordinateur (1600) de la revendication 1, comprenant en outre :
la réception, au niveau du dispositif de partie utilisatrice, d'informations indiquant l'état de santé de l'utilisateur, les informations indiquant l'état de santé de l'utilisateur ayant été fournies par le dispositif utilisateur (110).

10. Le procédé mis en œuvre par ordinateur (1600) de n'importe quelle revendication précédente, dans lequel le code pouvant être scanné est un code à réponse rapide QR.

11. Un système (100) comprenant :
au moins un processeur (310) ; et
une mémoire (320) couplée à l'au moins un processeur (310) et stockant des instructions qui, lorsqu'elles sont exécutées par l'au moins un processeur, amènent le système à effectuer le procédé (1600) de n'importe quelle revendication précédente.

12. Un programme informatique qui, lorsqu'il est exécuté sur au moins un processeur d'un système, est configuré pour amener le système à effectuer le procédé (1600) de n'importe laquelle des revendications 1 à 10.
